# EUROPEAN PATENT APPLICATION

(11) **EP 4 413 978 A2**
(43) Date of publication of application: **14.08.2024**
(21) Application number: 24020028.7
(22) Date of filing: 19.01.2024
(51) Int. Cl.: A61K 9/20, A61K 33/10, C02F 1/66, C02F 1/68, C02F 103/42

(54) **PROCESS FOR THE MANUFACTURE OF A TABLET**

(30) Priority: 19.01.2023 BE 202300002
(71) Applicant: De Herdt, Kris, 2500 Lier (BE)
(72) Inventor: De Herdt, Kris, 2500 Lier (BE)
(74) Representative: Theunis, Patrick

(57) **Abstract**

The present invention relates to a manufacturing process for a tablet.

The process comprises dry mixing of sodium bicarbonate and calcium carbonate powders in a weight ratio of 90 to 98 % of sodium bicarbonate and 10 to 2 % of calcium carbonate, at least 85 % of the particles of the sodium bicarbonate powder having a diameter up to 130 µm, the median diameter of the particles of the calcium carbonate powder being between 4 and 8 µm. Hereupon, whilst continue mixing, water is added to the mixture at a rate of between 5 to 15 % of the weight of the powders. Hereupon, mixing is continued until a homogeneous mixture has been obtained. Hereupon, the mixture is poured into molds and pressed under 2 to 6 kN/cm², followed by drying the pressed tablets.

The tablets so manufactured are used for the enhancement and resulting buffering action of water in amongst others swimming pools and swimming basins.

## Description

### Field of the invention

The present invention relates to a process for the manufacture of a tablet for enhancing the buffering action of water, more in particular of swimming pool water.

More in particular, the invention relates to a process for the manufacture of a tablet that when added to water, enhances its alkalinity and as a result hereof, enhances the buffering action of the (swimming pool) water.

The invention also relates to the tablet manufactured by such process, and the use of such tablet in a.o. swimming pools.

### Background of the invention

It is known that a good alkalinity of swimming pool water is important.

Such alkalinity is a measure of the acid buffering capacity of a liquid, for example water, or of the capacity of a solution to stabilize acids up to the point of equivalence of carbonate or bicarbonate. By such action, the alkalinity takes care of a stable pH value of a liquid. A correct pH value on turn takes care that disinfecting means such as chlorine can perform its function.

The alkalinity (TAC) or carbonate hardness indicates the concentration of carbonates and bicarbonates in water.

The TAC value is expressed in French degrees of mg/l CaCo3, 1 French degree corresponding to 10 mg/l of CaCO3.

The carbonate compounds fulfill a buffering action in the swimming pool water and as a result thereof guard the pH value of the water by neutralizing bigger variations of such pH value. The pH value can suddenly and drastically lower by atmospheric influences or the use of too many chemicals. It is important to periodically measure the alkaline value of swimming pool water with an appropriate test instrument.

A correct alkalinity value of the swimming pool water keeps the pH value stable and a correct pH value takes of a correct water-balance.

The alkalinity of swimming pool water may vary between 100 and 150 mg/liter.

The alkalinity value seldom is too high. If under exceptional circumstances the value exceeds the limit of 150 mg/liter, such value usually is automatically reduced by use of the swimming pool.

In case the value drops under the limit of 100 mg/liter, corrective action is required by adding the appropriate products.

To this end "Bayrol Alca-Plus" ^{®} can be used, producted by Bayrol, Chemische Fabrik GmbH, München, Germany, and available from the company Water Technics B.V., Mechelsesteenweg 277, 2500 Lier. This product is available as powder.

The disadvantage of the addition to swimming pool water of such product in powder form is that it can be blown away by the wind, such being highly uncomfortable for the handling person.

On top hereof, in view of the physical form of this powder, a correct dosage is difficult to measure and to keep for the user of the swimming pool. In order to ascertain the correct dosage, the correct amount of power needs to be determined, e.g. by weighing. In practical circumstances, this is not an easy task to be accomplished at the border of a swimming pool.

The annoying result of the above considerations is that when the product is added to the swimming pool water in the form of a powder, in most if not all cases either too much or too little is added. On top hereof, when "dropping" the product in powdery form to the swimming water, an unknown amount does not reach the swimming pool surface but is blown away by wind in the immediate surroundings of the swimming pool.

In practical circumstances, only in rare cases the correct amount of powder is dispersed into the swimming pool water. As a result, the desired buffering effect is seldom achieved. This further leads to supplementary additions of powder in the days following the first addition. In short, this way of working results in an annoying trial-and-error procedure, until the desired buffering action is finally reached, more or less by coincidence.

The aim of the inventors is to design a solution for the above-described annoying, impractical and inefficient operation.

### Summary of the invention

An object of the present invention is to overcome the above and further shortcomings by providing a process for the manufacture of a tablet for the enhancement of the buffering action of water.

The process according to the invention comprises the following steps:
a) dry mixing of sodium bicarbonate and calcium carbonate powders in a weight ratio of 90 to 98 % of sodium bicarbonate and 10 to 2 % of calcium carbonate, at least 85 % of the particles of the sodium bicarbonate powder having a diameter up to 130 µm, the median diameter of the particles of the calcium carbonate powder being between 4 and 8 µm;
b) whilst continue mixing, add water to the mixture at a rate of between 5 to 15 % of the weight of the powders;
c) continue mixing until a homogeneous mixture has been obtained;
d) pouring the mixture into molds and pressing under 2 to 6 k N/cm²;
e) drying the pressed tablets.

The present invention also relates to a tablet manufactured according to the above process.

In the tablet manufactured according to this process, the sodium bicarbonate acts as active compound or ingredient (for enhancing the alkalinity of water) and the calcium carbonate acts as binder.

According to a preferred embodiment, the tablet has a weight comprised between 100 and 1 000 gram.

The present invention also relates to the use of such tablet for the enhancement of the alkalinity, and as a result thereof, of the buffering action of water in swimming pools, swimming basins, ponds and recipients of rainwater.

More in particular, the invention relates to a manufacturing process for a tablet, a tablet manufactured according to such process, and the use of such tablet, as set forth in the attached claims.

The objects and advantages of the invention as set forth hereinafter are given by way of illustrative example, and such objects may be exemplary of one or more embodiments of the invention. Other desirable objectives and advantages inherently achieved by the disclosed invention may occur or become apparent to those skilled in the art.

### Statement and description of the invention

The invention is defined and characterized in the independent claims, while the dependent claims describe characteristics and specific features for preferred embodiments of the invention.

According to one aspect of the invention, the invention relates to a process for the manufacture of a tablet, the process comprising the following steps:
a) dry mixing of sodium bicarbonate and calcium carbonate powders in a weight ratio of 90 to 98 % of sodium bicarbonate and 10 to 2 % of calcium carbonate, at least 85 % of the particles of the sodium bicarbonate powder having a diameter up to 130 µm, the median diameter of the particles of the calcium carbonate powder being between 4 and 8 µm;
b) whilst continue mixing, add water to the mixture at a rate of 5 to 15 % of the weight of the powders;
c) continue mixing until an homogeneous mixture has been obtained;
d) pouring the mixture into molds and pressing under 2 to 6 k N/cm²;
e) drying the pressed tablets.

According to a preferred embodiment of the process according to the invention, the mixing under step a) lasts for 2-4 minutes per 25 kg of powders to be mixed.

According to a further preferred embodiment of the process according to the invention, the mixing under step c) lasts for 10-20 minutes per 25 kg of powders.

According to a still further preferred embodiment of the process according to the invention, the molding pressure of step d) amounts to 3-5 k N/cm². (kilo Newton per square centimeter).

According to a further preferred embodiment of the process according to the invention, the water to be added in step b) has a pH of around 7.3, a TAC value of around 150 ppm and a TH value of around 300 ppm. (TAC being a measure of the alkalinity of the water, TH being a measure of the hardness of the water.)

According to a still further preferred embodiment of the process according to the invention, the weight of the mixture poured into the molds is comprised between 100 and 1 000 gram, preferably between 250 and 850 gram, more preferably between 350 and 750 gram.

According to a still further preferred embodiment, the powder to be pressed to a tablet, comprises 500 gram of sodium bicarbonate, 26 gram of calcium carbonate and 75 ml of water. The resulting tablet then weighs 526 gram, comprising 95.06 % of sodium bicarbonate and 4.94 % of calcium carbonate.

The sodium bicarbonate to be used in the process according to the invention can be sourced from Solvay Operations France S.A., 2 Rue Gabriel Peri, 54110 Dombasle-sur-Meurthe, France. It is marketed under the trade name "Bicar Food 0/13 Sodium bicarbonate".

The calcium carbonate to be used in the process according to the invention can be sourced from S.A. Reverté Productos Minerales, Bellvei, Tarragona, Spain. It is marketed under the trade name "BL 50 S Reverté 7.12 µm".

The calcium carbonate used in the process according to the invention has a D50 value of between 4 and 8 µm, more preferably around 7 µm.

The D50 value is a measure of the particle size distribution, being the mass-median diameter, considered to the average particle size by mass.

So, according to a preferred embodiment of the process according to the invention, the following ingredients are used:
- 25 kg of sodium bicarbonate, obtainable from Solvay as Solvay Bicar Food, granulometry 0/13 µm;
- 1.3 kg of calcium carbonate, obtainable from Reverté as Reverté BL-50, food-grade, granulometry 6-10 µm;
- 3,671 of water, pH 7.3, TAC 150 ppm, TH 300 ppm.

The invention also relates to the tablet manufactured according to the process as described supra.

The tablet manufactured according to such process, comprises 90 to 98 % of sodium bicarbonate, acting as active ingredient, and 10-2 % of calcium carbonate, acting as binder.

According to a still further aspect of the invention, the tablet comprises sodium bicarbonate and calcium carbonate in a weight ratio of 500/26.

According to a still further aspect of the invention, the weight of the tablet is approximately 500 gram.

The invention also relates to the use of the tablet manufactured according to such process.

According to one aspect of the invention, the tablet is used for enhancing the alkalinity of water in swimming pools, swimming basins, ponds and recipients of rainwater.

According to a further aspect of the invention, the tablet as dissolved in water, acts as chemical buffer.

The unexpected advantage of the process according to the invention is that the combination of the various features of such process, i.a. the granulometry of both ingredients, the relative amount of both ingredients, the sequence of steps of the process, and the molding pressure applied during step d) of the process, are so tuned or set that the tablet resulting from such manufacturing process, slowly dissolves in water.

More in particular, the inventors have noted that the above combination of features yields a tablet that when added to (swimming pool) water smoothly and evenly dissolves over a period of 24 hours, when a flow of 6 m³/hour in the skimmers of a swimming pool is applied. The latter corresponding to the usual flow rate applied in swimming pools.

So according to the manufacturing process of the invention, a tablet is obtained that when added to (swimming pool) water, is not blown away in an uncontrolled manner as is the case with the powders of the prior art, and enhances the alkalinity of the water in an utmost efficient way as it gradually, slowly and evenly dissolves into the water over a period of approximately 24 hours, under the usual flow conditions as applied in the (skimmers) of a swimming pool.

The (pure) sodium bicarbonate powder known as such according to the state of the art is unsuitable for being molded under pressure in the form of a tablet. In order to bind the powdery product in the form of a tablet, a binder is required. Tests as performed by the inventors have revealed that in case the pure sodium bicarbonate powder is molded under pressure under the addition of some water, a tablet-like product is obtained. However, when such tablet is added to the water of a swimming pool or a swimming basin, it appears that already after one hour, the tablet is completely dissolved, the latter being too fast.

Further tests have also revealed that the use of calcium hydroxide as binder, in combination with sodium bicarbonate, does not yield satisfying results for the resulting tablet.

In order for the tablet to dissolve over time in a smooth and controlled manner, the incorporation of a suitable binder in the tablet is an absolute requirement. This role can be accomplished by calcium carbonate. The smooth, regular, even and controlled solution of the tablet in water, enhances in a substantive manner the efficiency of the tablet according to the invention.

The molding pressure applied during the manufacturing process of the tablet also has an effect on the rate of solution of the tablet in water. A molding pressure of for example between 3 to 6 kN/cm², more preferably between 4 to 5 kN/cm², yields a tablet that suits the intended purpose.

The higher the molding pressure, the less water needs to be added to the mixture. However, from the point of view of the desired solubility in water, the molding pressure is not the key determining factor, but the amount of binder, and in particular the kind of calcium compound that is used. As set forth supra, tests have revealed that in combination with sodium bicarbonate as active ingredient, calcium carbonate as binder yields the desired effect.

The invention also relates to the use of said tablet for the enhancement of the alkalinity, and as a result thereof, of the buffering action of water in swimming pools, swimming basins, ponds and recipients of rainwater.

According to a further preferred embodiment of the invention, the tablet as dissolved in the water as treated, acts as a chemical buffer.

According to a still further preferred embodiment of the invention, the tablet is used for the re-mineralization of filtered water, more in particular osmosis water.

### Advantages of the tablet according to the invention

As set forth supra, one of the advantages of the use of the tablet according to the invention, is that upon addition of the tablet to e.g. the water of a swimming pool, the contents of the tablet will not be blown away into the air in an uncontrolled manner.

A further advantage of the tablet according to the invention is its ease of use for the customer, user of a swimming pool or swimming basin.

This advantage directly results from the ease of calculation of the amount of tablets to be added to the water, as well as the certainty that irrespective of the atmospheric conditions, absence or presence of wind, a well-determined amount of product will be added to the water to be treated.

When the powders known from the state of the art for enhancing the alkalinity of water are used, all consisting of pure sodium bicarbonate, there are almost no users that make use of a weighing machine.

According to the guidelines for dosing, the addition of 180 gram per 10 m3 of water enhances the alkalinity by 10 ppm. This is a quite complicated calculation for most non-professional customers. However, if a tablet comprising 450 gram of active ingredient (sodium bicarbonate) is made available, the calculation is easily made as such tablet will enhance the alkalinity of 25 m3 of water with 10 ppm. An (average) swimming pool comprising 50 cubic meter of water will need two tablets in order to enhance the alkaline value with 10. And this result is predictable and certain as the customer does not need to calculate and weigh the appropriate amount of powder any more.

Finally, there is a substantive and unexpected advantage resulting from the addition of sodium bicarbonate to swimming pool water in a form as contained in a pressed tablet: in this form, the sodium bicarbonate is slowly, smoothly and evenly dissolved in the water treated. The efficiency of the product is substantively enhanced in this manner. The risk of stains in the swimming pool is reduced and the effect of the product on the water treated is optimal as the intake of the ingredients of the tablet by the water treated occurs over time in a smooth and controlled manner.

## Claims

1. Process for the manufacture of a tablet, comprising the following steps:
a) dry mixing of sodium bicarbonate and calcium carbonate powders in a weight ratio of 90 to 98 % of sodium bicarbonate and 10 to 2 % of calcium carbonate, at least 85 % of the particles of the sodium bicarbonate powder having a diameter up to 130 µm, the median diameter of the particles of the calcium carbonate powder being between 4 and 8 µm;
b) whilst continue mixing, add water to the mixture at a rate of between 5 to 15 % of the weight of the powders;
c) continue mixing until an homogeneous mixture has been obtained;
d) pouring the mixture into molds and pressing under 2 to 6 k N/cm²;
e) drying the pressed tablets.

2. Process according to claim 1, wherein the mixing under step a) lasts for 2-4 minutes per 25 kg of powders to be mixed.

3. Process according to claim 1 or 2, wherein the mixing under step c) lasts for 10-20 minutes per 25 kg of powders.

4. Process according to any of the preceding claims, wherein the molding pressure of step d) amounts to 3-5 k N/cm².

5. Process according to any of the preceding claims, wherein the weight of the mixture poured into the molds is comprised between 100 and 1 000 gram, preferably between 250 and 850 gram, more preferably between 350 and 750 gram.

6. Tablet manufactured according to any of the preceding claims.

7. Use of the tablet according to claim 6 for the enhancement of the alkalinity of water in swimming pools, swimming basins, ponds and recipients of rainwater.

8. Use of the tablet according to claim 6, the tablet as dissolved in water acting as chemical buffer.

9. Use of the tablet according to claim 6 for the remineralization of filtered water, more in particular, osmosis water.
